# EUROPEAN PATENT APPLICATION

(11) **EP 4 480 534 A1**
(43) Date of publication of application: **25.12.2024**
(21) Application number: 23756245.9
(22) Date of filing: 07.02.2023
(51) Int. Cl.: A61N 2/04

(54) **MEDICAL DEVICE**

(30) Priority: 18.02.2022 JP 2022023633
(71) Applicant: Nipro Corporation, Settsu-shi, Osaka 566-8510 (JP)
(72) Inventor: OGASAWARA, Hitoshi, Chuo-shi, Yamanashi 409-3801 (JP); MURAKAMI, Motoaki, Chuo-shi, Yamanashi 409-3801 (JP)
(74) Representative: Mewburn Ellis LLP
(86) International application number: PCT/JP2023/003981
(87) International publication number: WO 2023/157718

(57) **Abstract**

The present invention is to provide a medical apparatus superior in drip-proofness (IPX), electrostatic discharge/surge (ESD) countermeasure, and electromagnetic susceptibility (EMS) countermeasure, and with which there can be achieved a treatment effect required to treat pain or the like in an affected area. The medical apparatus is equipped with an apparatus main body having an inlet for an inlet connector to be connected thereto, and includes:
a gasket that is interposed between an inlet opening section provided at the inlet and a casing opening section for inlet connector connection provided at the apparatus main body, and is configured to allow the inlet to conform with the shape of the inlet connector.

## Description

### Technical Field

The present invention relates to a medical apparatus. Particularly, the present invention relates to a medical apparatus that is superior in medically unique countermeasures such as a drip-proof countermeasure, electrostatic discharge/surge (ESD) countermeasure, and electromagnetic susceptibility (EMS) countermeasure, and is used to treat pain in an affected area of a living body by producing a biostimulation signal wave and stimulating the cells in the affected area as a result of irradiating the affected area with a magnetic field that is generated at a coil via such signal wave.

### Background Art

As an apparatus for treating pain in an affected area of a living body by stimulating the cells in the affected area as a result of irradiating the affected area with a magnetic field, there is conventionally known, for example, an apparatus disclosed in Patent Literature 1. This treatment apparatus is configured in such a manner that a high-frequency coil and a low-frequency coil are respectively formed into a helical or looped shape and are received in a housing along with an oscillation circuit and a battery, which allows the apparatus to be carried around.

Further, this treatment apparatus treats pain in an affected area in the following manner. That is, magnetic fields are respectively generated at these high-frequency coil and low-frequency coil via a high-frequency signal and a low-frequency signal with certain frequencies that are outputted from the oscillation circuit. By placing the housing on an affected area of a living body, the affected area will be irradiated with the magnetic fields to have the cells in the affected area stimulated, whereby this stimulation promotes the production of the neurotrophic factors in the cells of the affected area and whereby promotes the repair, growth, differentiation, and proliferation of these cells to treat pain in the affected area.

Moreover, Patent Literature 2 discloses a system or the like for electromagnetic induction treatment that is configured as follows. The system includes one or more probes that are ergonomic or contoured to a specific region of the body. This probe includes one or more conductive coils that are configured to generate an electromagnetic field or magnetic field focused on a target nerve, muscle or other body tissues in proximity to the coil. One or more sensors are utilized to detect stimulation and provide feedback about the efficacy of the applied electromagnetic induction treatment. A controller is adjustable to vary a current through the coil so as to adjust the magnetic field focused upon the target nerve, muscle or other body tissues based on the feedback provided by the sensor or the patient.

### Citation List

### Patent Literature

Patent Literature 1: WO2008/056414
Patent Literature 2: Japanese Unexamined Patent Application Publication (Translation of PCT Application) No.2013-508119

### Summary of Invention

### Technical Problem

However, the problem with the apparatus disclosed in Patent Literature 1 is that since the high-frequency output coil and the low-frequency output coil are received in the housing together with the oscillation circuit and the battery, the housing needs to be placed on an affected area in order for the affected area to be irradiated with the magnetic fields generated at these coils, thereby making the housing bulky and cumbersome, or even causing the device to fall off the affected area after being caught on clothes.

Further, the apparatus disclosed in Patent Literature 1 does not have an inlet; and the drip-proofness (IPX) or the like in the housing is not taken into consideration despite the housing receiving the high-frequency output coil, the low-frequency output coil, the oscillation circuit, and the battery.

The sensor disclosed in Patent Literature 2 is to detect the electric conduction of the nerves stimulated by electromagnetic treatment, where water content and sweat emitted from the skin of a patient can be appropriately ejected outside from a vent hole while an electrode carrier is placed on the skin of the patient. However, the sensor disclosed in Patent Literature 2 does not have an inlet; and no consideration is given to the drip-proofness (IPX) of the sensor as well as the influence of electrostatic discharge that is caused by contact between the human body and the magnetic treatment apparatus.

In the meantime, drip-proofness is one of the properties required for a medical apparatus. Among medical apparatuses, magnetic treatment apparatuses are in particular used in contact with the human body. Therefore, there is a possibility that water and moisture may enter the interior of a magnetic treatment apparatus. A magnetic treatment apparatus without a drip-proof property will cause ground faults and electrical leakage accidents as the insulating property of the apparatuses deteriorates due to the water that has entered the interior. Particularly, a magnetic treatment apparatus with an inlet has an opening section for connecting an inlet connector or the like, which tends to absorb water and moisture from such opening section. That is, a medical apparatus such as an inlet-equipped magnetic treatment apparatus is required to possess a drip-proofness (IPX) capable of preventing water content from entering an interior of the apparatus and thereby withstanding a long-term use of the magnetic treatment apparatus.

On the other hand, contact between the human body and a medical apparatus such as a magnetic treatment apparatus generates static electricity, and a phenomenon known as electrostatic discharge will occur. The voltage generated by this phenomenon (electrostatic discharge/surge (ESD; Electro-Static Discharge)) is a high voltage of several thousand volts, and the high voltage pulse penetrates into the interior of a treatment apparatus, causing malfunction and/or destruction of the IC circuit built in a medical apparatus such as the magnetic treatment apparatus. In order to prevent such static electricity entry into the interior of a magnetic treatment apparatus, it is necessary to suppress and eliminate electrostatic discharge/surge (ESD). Further, a magnetic treatment apparatus is susceptible to electromagnetic interference. Interference caused by the susceptibility of a medical apparatus such as a magnetic treatment apparatus to electromagnetic interference will adversely affect the performance of the apparatus.

That is, a medical apparatus requires an electrostatic discharge (ESD)/surge (ESD) countermeasure for the purpose of suppressing and eliminating electrostatic discharge/surge (ESD), and an electromagnetic susceptibility (EMS; Electromagnetic Susceptibility) countermeasure. Electrostatic discharge/surge (ESD) countermeasure and electromagnetic susceptibility (EMS) countermeasure are required for all parts and locations of medical apparatuses where the human body comes into contact with. Specifically, as parts and locations requiring electrostatic discharge/surge (ESD) and electromagnetic susceptibility (EMS) countermeasures, there may be listed, for example, locations such as USB and LAN output terminals where the user plugs and unplugs an inlet connector; and operation buttons on a magnetic treatment apparatus. For this reason, there have been established standards for electrostatic discharge/surge (ESD) and electromagnetic susceptibility (EMS) countermeasures that a medical apparatus such as a magnetic treatment apparatus must meet.

The present invention was made in view of these technical issues, and it is an object of the present invention to provide a medical apparatus superior in drip-proofness (IPX), electrostatic discharge/surge (ESD) countermeasure, and electromagnetic susceptibility (EMS) countermeasure, and with which there can be achieved a treatment effect required to treat pain or the like in an affected area.

### Solution to Problem

In order to solve the above problems, the medical apparatus of the present invention is a medical apparatus equipped with an apparatus main body having an inlet for an inlet connector to be connected thereto, where the apparatus is characterized by having:
a gasket that is interposed between an inlet opening section provided at the inlet and a casing opening section for inlet connector connection provided at the apparatus main body, and is configured to allow the inlet to conform with the shape of the inlet connector.

Further, with regard to the medical apparatus of the present invention, it is considered that more preferred solutions can be brought when, for example,
(a) the gasket includes:
   a gasket first sealing portion that tightly engages with an end surface of an outer circumferential portion of the inlet connector by pressure-contacting the inlet connector with the inlet; and
   a gasket second sealing portion that is provided on an inner wall of an opening section of the gasket and tightly engages with an inner circumferential portion side surface of the inlet connector by pressure-contacting the inlet connector with the inlet;
(b) there are provided multiple said gasket second sealing portions facing against the inner wall of the opening section of the gasket;
(c) the medical apparatus has a waterproof rating (IPX) of 1 or higher;
(d) the medical apparatus has an electrostatic discharge resistance even after static electricity has been discharged according to an electrostatic discharge test;
(e) the electrostatic discharge resistance is an air electrostatic discharge resistance of ±15 kV or higher; and
(f) the medical apparatus further includes a probe formed separately from the apparatus main body and having a coil, wherein the medical apparatus treats pain in an affected area of a living body by producing a biostimulation signal wave and stimulating cells and nerves in and around the affected area as a result of irradiating the affected area with a magnetic field for affected area stimulation that is generated at the coil via the biostimulation signal wave.

### Advantageous Effects of Invention

The present invention provides a medical apparatus equipped with an inlet superior in drip-proofness (IPX), electrostatic discharge/surge (ESD) countermeasure, and electromagnetic susceptibility (EMS) countermeasure. That is, according to the medical apparatus of the present invention, due to the gasket allowing the inlet to conform with the shape of the inlet connector, the insulation performance of the apparatus will not deteriorate as there will be no water entry into the interior of the apparatus main body of the medical apparatus, and there will thus be no ground faults and electrical leakage accidents caused thereby.

In addition, in the medical apparatus of the present invention, due to the gasket allowing the inlet to conform with the shape of the inlet connector, the sealing performance of the inlet portion is ensured whereby powdery dust will not adhere to a drive part inside the apparatus main body, and malfunction of the medical apparatus will not occur accordingly. That is, the medical apparatus of the present invention is superior in drip-proofness (IPX), electrostatic discharge/surge (ESD) countermeasure, and electromagnetic susceptibility (EMS) countermeasure.

### Brief Description of Drawings

[FIG. 1] is a perspective view showing the entire appearance of a magnetic treatment apparatus of one embodiment of the present invention.
[FIG.2] is a front view showing the appearance of an apparatus main body of the magnetic treatment apparatus of the above embodiment.
[FIG.3] is an enlarged cross-sectional view showing how an inlet and gasket provided on the magnetic treatment apparatus of the above embodiment interact with an inlet connector that is to be connected to the inlet.
[FIG.4] is a set of schematic diagrams showing the structure of the gasket of the magnetic treatment apparatus of the above embodiment, in which FIG.4(a) is a front perspective view, FIG.4(b) is a rear perspective view, and FIG.4(c) is a A-A cross-sectional view.
[FIG.5] is a set of schematic diagrams showing states before and after connecting an inlet connector to the inlet built in the magnetic treatment apparatus of the above embodiment, in which FIG.5(a) is a schematic diagram showing a state before connecting the inlet connector to the inlet, and FIG.5(b) is a schematic diagram showing a state after connecting the inlet connector to the inlet.

### Description of Embodiments

A medical apparatus of this embodiment will be described in detail hereunder with reference to drawings. Each drawing is schematic and may be different from the actual device. Further, the embodiment shown below is an example of an apparatus embodying the technical concept of the present invention and is not to limit the configuration of the present invention to those shown below. That is, various modifications can be made to the technical concept of the present invention within the technical scope described in the claims.

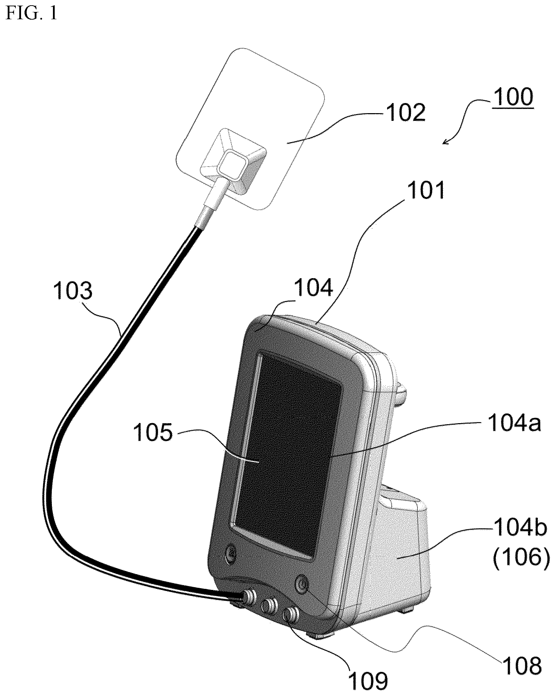

FIG. 1 is a perspective view showing the entire appearance of a magnetic treatment apparatus 100 as an example of a medical apparatus of one embodiment according to the present invention. Here, a medical apparatus is an apparatus whose production and distribution are regulated by the Act on Pharmaceutical and Medical Devices, examples of which may include a magnetic treatment apparatus, a blood glucose meter, a home electric potential treatment apparatus, a home massage apparatus, and an electronic blood pressure meter. In this embodiment, as one such example, described is a magnetic treatment apparatus as a medical device that is equipped with an apparatus main body having an inlet allowing an inlet connector to be connected thereto. As shown in FIG. 1, the magnetic treatment apparatus 100 according to the embodiment has an apparatus main body 101, a probe 102, a communication cable 103 for connecting the probe 102 to the apparatus main body 101, and a power cable that is not shown and is detachably plugged into and attached to the apparatus main body 101.

The magnetic treatment apparatus 100 of this embodiment is a magnetic treatment apparatus that treats pain in an affected area of a living body by producing biostimulation signal waves and stimulating the cells in the affected area as a result of irradiating the affected area with magnetic fields that are generated at coils via such biostimulation signal waves; the magnetic treatment apparatus 100 is also superior in drip-proofness (IPX), electrostatic discharge/surge (ESD) countermeasure, and electromagnetic susceptibility (EMS) countermeasure. The apparatus main body 101 of the magnetic treatment apparatus 100 has, for example, a signal wave output part that generates and outputs a biostimulation signal wave of a first frequency. The apparatus main body 101 has a resin casing 104; and a touch input-type (capacitive) display 105 that is housed obliquely upward in the casing 104 and is exposed from an opening section 104a of the front surface of the casing 104. Disposed inside of a protruding part 104b on the rear lower side of the casing 104 is a battery 106 for supplying a power source required to put the magnetic treatment apparatus 100 into operation. Here, the specifications of the battery 106 can be appropriately configured.

The magnetic treatment apparatus 100 has the probe 102 formed separately from the apparatus main body 101 and having, as one of the coils, a first coil that is connected to the signal wave output part through the communication cable 103 and is supplied with the biostimulation signal wave of the first frequency which is outputted from the signal wave output part. The probe 102 is a member for treating pain in an affected area of a living body by generating a magnetic field(s) when placed on the affected area and irradiating the affected area with such magnetic field at the time of using the magnetic treatment apparatus 100 of this embodiment. It will suffice if the magnetic treatment apparatus 100 has at least one probe 102; the number of the probes 102 may be increased as appropriate. Here, the communication cable 103 may be configured integrally with the apparatus main body 101 and the probe 102, configured in a detachable (inletting) manner with respect to the apparatus main body 101 and the probe 102, or configured separately therefrom.

In the magnetic treatment apparatus 100 of this embodiment, the probe 102 is provided as a component separate from the apparatus main body 101. Thus, by placing only the probe 102 on an affected area of a living body, the affected area can be irradiated with, for example, the magnetic field generated at the first coil. Here, a signal wave generation part and a power supply part are not housed in the probe 102. For this reason, the probe 102 can be designed in a more compact fashion as compared to the device main body 101. As a result, when using the magnetic treatment apparatus 100, the probe 102 will not become bulky and fall off the affected area after being caught on clothes.

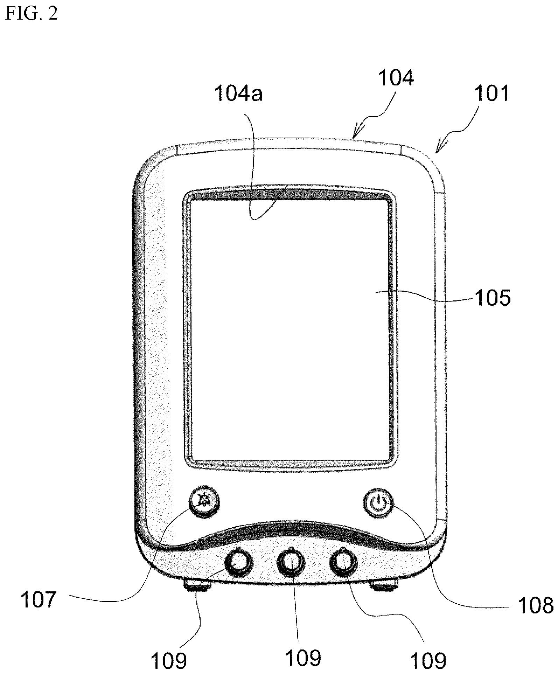

FIG.2 is a front view showing the appearance of the apparatus main body of the magnetic treatment apparatus of this embodiment. As shown in FIG.2, an alarm stop button 107 and a power switch button 108 are provided on the left and the right side below the opening section 104a on the front surface of the casing 104 of the apparatus main body 101. Even below the alarm stop button 107 and the power switch button 108, there are provided with three horizontally aligned sockets 109 for plugging in the communication cable 103 so that the sockets 109 enable three probes 102 to be connected to the apparatus main body 101. Here, the number of the sockets 109 can be appropriately selected depending on the number of the probes 102 installed.

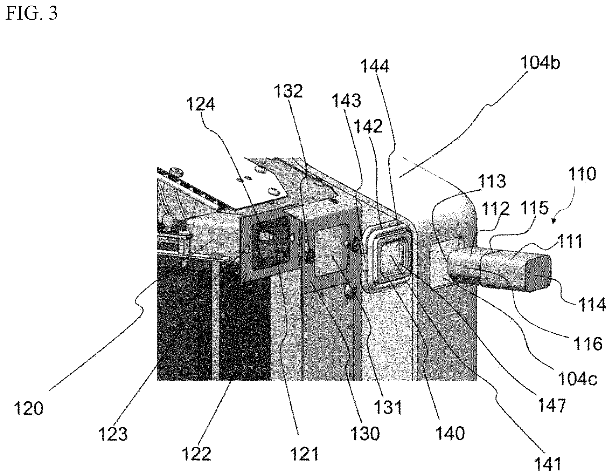

FIG.3 is an enlarged cross-sectional view showing how the inlet and a gasket provided on the magnetic treatment apparatus of this embodiment interact with the inlet connector that is to be connected to the inlet from outside. A casing opening section for inlet connector connection 104c for connecting an inlet connector 110 from outside the apparatus main body 101 is provided on the protruding part 104b of the rear lower area of the casing 104 of the magnetic treatment apparatus 100 of this embodiment. The inlet connector 110 that is connected from outside the apparatus main body 101 is composed of an inlet connector outer circumferential portion 111 and an inlet connector inner circumferential portion 112. By being inserted toward the casing opening section for inlet connector connection 104c, the inlet connector 110 will be connected to an inlet 120 built in the protruding part 104b.

Provided inside the protruding part 104b is the inlet 120 for the inlet connector 110 to be connected thereto from outside the apparatus main body 101. Further, provided inside the protruding part 104b is a gasket 140 configured to allow the inlet 120 to conform with the shape of the inlet connector 110. The gasket 140 has a gasket first sealing portion 144 that tightly engages with an inlet connector step portion end surface 115 by pressure-contacting the inlet connector 110 with the inlet 120; and a gasket second sealing portion 147 that is provided on the inner wall of a gasket opening section 141 and tightly engages with the inlet connector inner circumferential portion 112 by pressure-contacting the inlet connector 110 with the inlet 120.

The gasket 140 is interposed between an inlet opening section 121 provided at the inlet 120 and the casing opening section for inlet connector connection 104c provided at the device main body 101. When connecting the inlet connector 110 to the inlet 120 from outside the apparatus main body 101, from the outer side of the apparatus main body 101 toward the inner side of the inlet 120, engagement of members will occur in an order of the inlet connector 110, the protruding part 104b of the casing 104, the gasket 140, an inner substrate 130, and the inlet 120, whereby a sealed condition of a space established between the inlet 120 and the inlet connector 110 will be maintained. In this way, the magnetic treatment apparatus 100 of this embodiment is configured in such a manner that provided inside the protruding part 104b is the gasket 140 designed to allow the inlet 120 to conform with the shape of the inlet connector 110, thereby making it possible to ensure the sealability between the inlet 120 and the inlet connector 110. As a result, the magnetic treatment apparatus of this embodiment can employ a sufficient drip-proof structure and is superior in electrostatic discharge/surge (ESD) countermeasure and electromagnetic susceptibility (EMS) countermeasure.

The inlet connector 110 that is to be connected from outside the apparatus main body 101 is composed of the inlet connector outer circumferential portion 111 and the inlet connector inner circumferential portion 112. An inlet connector inner circumferential portion end surface 113 of the inlet connector inner circumferential portion 112 is designed to be smaller than an end surface 114 of the inlet connector outer circumferential portion 111 so as to match the shape of the opening section of the inlet 120. Thus, since the size of the inlet connector outer circumferential portion 111 and the size of the inlet connector inner circumferential portion 112 are different, the inlet connector step portion end surface 115 is established at a joined part between the inlet connector outer circumferential portion 111 and the inlet connector inner circumferential portion 112.

Here, the shape of the inlet connector inner circumferential portion end surface 113 of the inlet connector inner circumferential portion 112 is the shape of the inlet opening section 121 through which a normal inlet connector 110 is to be connected to the inlet 120. It will suffice if the shape of the inlet connector inner circumferential portion end surface 113 of the inlet connector inner circumferential portion 112 is designed to be smaller than the end surface 114 of the inlet connector outer circumferential portion 111; the inlet connector inner circumferential portion end surface 113 may have any of, for example, a substantially rectangular shape, a substantially trapezoidal shape, a substantially circular shape, and a substantially oval shape. The shape of the end surface 114 of the inlet connector outer circumferential portion 111 may be identical to or different from the shape of the inlet connector inner circumferential portion end surface 113 of the inlet connector inner circumferential portion 112; the end surface 114 may have any of, for example, a substantially rectangular shape, a substantially trapezoidal shape, a substantially circular shape, and a substantially oval shape.

The inlet 120 has the inlet opening section 121 and an inlet flange 122 that is provided on the outer edge of the inlet opening section 121. Formed on the inlet flange 122 is a fixing tool hole 123 for attaching the inner substrate 130. Here, installed inside the inlet 120 is an inlet electric terminal 124 used to electrically connect the inlet connector 110 and the inlet 120.

The inner substrate 130 has, in the inner part thereof, an inner substrate opening section 131 allowing the inlet connector inner circumferential portion 112 of the inlet connector 110 to penetrate therethrough. The shape of the inner substrate opening section 131 is identical to the inlet connector inner circumferential portion end surface 113 of the inlet connector inner circumferential portion 112. The inner substrate 130 is to be attached to the inlet flange 122 by a fixing tool screw(s) 132 via through holes formed on the inner substrate. As a result, the inner substrate 130 can tightly engage with and be fixed to the inlet 120 without forming a gap therebetween.

The gasket 140 is attached to the surface of the inner substrate 130. The gasket 140 has, in the inner part thereof, the gasket opening section 141 allowing the inlet connector inner circumferential portion 112 of the inlet connector 110 to penetrate therethrough. A gasket outer edge portion 142 is provided on the outer edge of the gasket 140. Formed on the back surface of the gasket outer edge portion 142 is a fixing tool screw receiving portion 143 for receiving the fixing tool screw 132 that protrudes from the surface of the inner substrate 130. The fixing tool screw 132 has a convex shape protruding in the casing 104 direction of the protruding part 104b with respect to the inner substrate 130. The fixing tool screw 132 engages with the fixing tool screw receiving portion 143. As a result, the gasket 140 can engage with and be fixed to the inner substrate 130 without forming a gap therebetween.

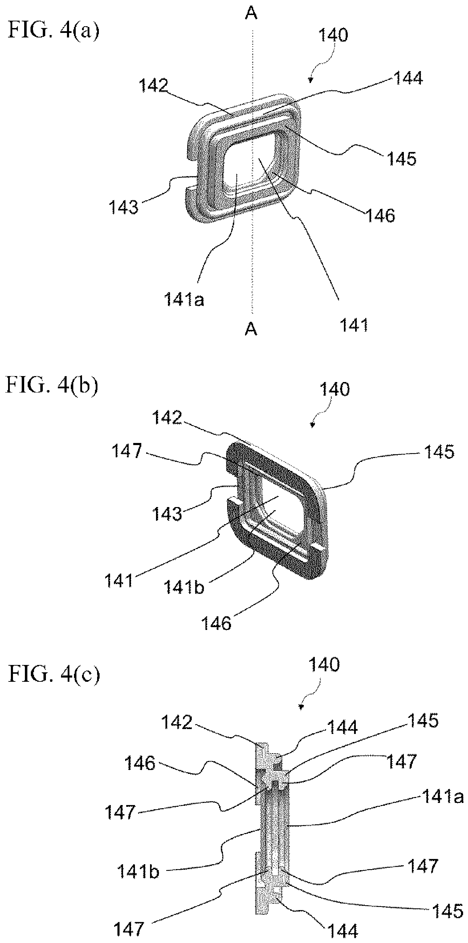

FIG.4 is a set of schematic diagrams showing the structure of the gasket of the magnetic treatment apparatus of this embodiment. FIG.4(a) is a front perspective view, and FIG.4(b) is a rear perspective view. FIG.4(c) is a A-A cross-sectional view. As shown in FIGs.4(a) and 4(b), the gasket 140 has the gasket first sealing portion 144 and a gasket front surface convex portion 145 that protrude in the casing 104 direction of the protruding part 104b. The gasket first sealing portion 144 and the gasket front surface convex portion 145 are formed into the shape of a substantially rectangular frame, and have the gasket opening section 141 formed in the inner part thereof. That is, from the inner substrate 130 toward the casing 104 direction of the protruding part 104b, the gasket 140 has parts formed in an order of the gasket outer edge portion 142, the gasket first sealing portion 144, and the gasket front surface convex portion 145. The gasket first sealing portion 144 is formed to protrude in the casing 104 direction of the protruding part 104b. The outer frame shape of the gasket front surface convex portion 145 is identical to the shape of the casing opening section for inlet connector connection 104c that allows the inlet connector 110 to penetrate therethrough and connect the inlet 120. Thus, the gasket front surface convex portion 145 can be engaged with the casing opening section for inlet connector connection 104c. By engaging the gasket front surface convex portion 145 with the casing opening section for inlet connector connection 104c, the casing 104 of the protruding part 104b can tightly engage with and be fixed to the gasket 140 without forming a gap therebetween.

From the inlet 120 toward the casing 104 direction of the protruding part 104b, the gasket 140 has parts formed in the order of the gasket outer edge portion 142, the gasket first sealing portion 144, and the gasket front surface convex portion 145, and has the gasket opening section 141 formed in the inner part thereof. Formed at the gasket opening section 141 are a gasket first opening section 141a allowing the inlet connector outer circumferential portion 111 of the inlet connector 110 to penetrate therethrough; and a gasket second opening section 141b allowing the inlet connector inner circumferential portion 112 to penetrate therethrough. That is, the gasket opening section 141 is configured as a double structure by the gasket first opening section 141a and the gasket second opening section 141b.

In other words, the gasket opening section 141 has two opening sections allowing the inlet connector outer circumferential portion 111 and inlet connector inner circumferential portion 112 to penetrate therethrough, the inlet connector outer and inner circumferential portions 111 and 112 corresponding to different end surfaces of the inlet connector 110. The gasket first opening section 141a and the gasket second opening section 141b are divided by a gasket opening section dividing part 146 provided on the inner wall of the gasket opening section 141. By employing an opening section of the same shape as the inlet connector inner circumferential portion end surface 113 of the inlet connector inner circumferential portion 112, the gasket opening section dividing part 146 forms the gasket second opening section 141b.

FIG.4(c) is the A-A cross-sectional view of the gasket. As shown in FIG.4(c), the gasket 140 has the gasket first sealing portion 144 and the gasket second sealing portion 147. The gasket first sealing portion 144 is formed into the shape of a frame and is formed all over the gasket opening section 141 along the circumferential direction thereof. Further, the cross-sectional surface of the gasket first sealing portion 144 has a shape protruding in the casing direction of the casing protruding part 104b. The gasket first sealing portion 144 pressure-contacts with the inlet connector step portion end surface 115. When the inlet connector outer circumferential portion 111 is press-fitted toward the direction of the inlet 120 by passing through the gasket first opening section 141a, the inlet connector step portion end surface 115 will tightly engage with the gasket first sealing portion 144 without forming a gap therebetween. As a result, the gap formed between the inlet connector outer circumferential portion 111 and the gasket outer edge portion 142 can be completely sealed by the gasket first sealing portion 144.

That is, in the case of the apparatus main body 101 of the magnetic treatment apparatus 100 of this embodiment, since the gasket first sealing portion 144 is provided all over the gasket opening section 141 along the circumferential direction thereof, the gap formed between the inlet connector outer circumferential portion 111 and the gasket outer edge portion 142 can be sealed. Here, the inlet connector step portion end surface 115 is pressed against and fixed to the gasket opening section dividing part 146 that is provided inside the gasket opening section 141.

In this way, in the case of the apparatus main body 101 of the magnetic treatment apparatus 100 of this embodiment, since the gasket 140 has the gasket first sealing portion 144, water content such as water and moisture can be effectively prevented from entering the gap that is located between the inlet connector outer circumferential portion 111 and the gasket outer edge portion 142 and is formed along the circumferential direction of the gasket 140. That is, in the case of the magnetic treatment apparatus 100 of this embodiment, the gasket first sealing portion 144 allows the gap formed along the circumferential direction of the gasket 140 to be completely sealed, whereby water content can be effectively prevented from entering this gap.

As a result, the magnetic treatment apparatus 100 of this embodiment is superior in drip-proofness. Here, drip-proofness is defined as protection against dripping water. Further, drip-proofness is evaluated by the waterproof rating (IPX) which is a set of evaluation criteria that are called "IP (International Protection) codes" and are stipulated by IEC (International Electro-technical Commission). The waterproof rating (IPX) rates protective performance of precision machineries and apparatuses such as magnetic treatment apparatuses against water and solids.

The waterproof rating (IPX) is classified into 9 categories from 0 to 8. The contents of protection and testing method in each category is as follows. Here, tap water of normal temperature is used as the water for testing, and it is verified whether or not the apparatus is able to function normally as a magnetic treatment apparatus after testing. The waterproof rating (IPX) 1 specifies protection against dripping water. This is a waterproof property where no water ingress is observed with the magnetic treatment apparatus after using a drip testing device to vertically deliver water by drops at a rate of 1 (mm/min) for 10 min. The waterproof rating (IPX) 2 specifies protection against falling water when tilted to 15°. This is a waterproof property where no water ingress is observed with the magnetic treatment apparatus after performing a watering test in which a drip testing device is used to deliver water by drops at a rate of 3 (mm/min) on the magnetic treatment apparatus tilted to 15°, and the test is performed for a total of 10 min with 2.5 min each from each direction.

The waterproof rating (IPX) 3 specifies protection against spraying water.

Specifically, this is a waterproof property where no water ingress is observed with the magnetic treatment apparatus after performing watering for 10 min from the vertical direction to an angle(s) of 60° on both sides with a water volume of 0.07 (L/min) per each watering port, using a watering device equipped with oscillating tubes or watering nozzles. The waterproof rating (IPX) 4 specifies protection against splashing water. This is a waterproof property where no water ingress is observed with the magnetic treatment apparatus after performing watering for at least 5 min from any angle with the water volume of 0.07 (L/min) per each watering port, using the abovementioned watering device. The waterproof rating (IPX) 5 specifies protection against jetted water stream. This is a waterproof property where no water ingress is observed with the magnetic treatment apparatus after performing watering, from any direction, for a total of 3 min or longer with 1 min each for each 1 m² of the outer cover surface area, using a water stream of 12.5 (L/min) discharged from a watering nozzle of *φ* 6.3 (mm). The waterproof rating (IPX) 6 specifies protection against waves. This is a waterproof property where no water ingress is observed with the magnetic treatment apparatus after performing watering, from any direction, for a total of 3 min or longer with 1 min each for each 1 m² of the outer cover surface area, using a water stream of 100 (L/min) discharged from a watering nozzle of ϕ 12.5 (mm). The waterproof rating (IPX) 7 specifies protection against water immersion. This is a waterproof property where no water ingress is observed with the magnetic therapy device after submerging the device in a water tank with a depth of 1 m for 30 min. The waterproof rating (IPX) 8 specifies protection against continual water submersion. This is a waterproof property where no water ingress is observed with the magnetic treatment apparatus after submerging the apparatus in a water tank with a depth of 1 m for more than 30 min. Here, a magnetic treatment apparatus satisfying the waterproof rating (IPX) 8 has to have a completely sealed structure in principle. In the case of the magnetic treatment apparatus 100 of this embodiment, due to the gasket first sealing portion 144, the gap formed in the circumferential direction of the gasket 140 is completely sealed, thereby allowing the apparatus of this embodiment to possess a drip-proofness of at least the waterproof rating (IPX) 1 or higher. Further, in the case of the magnetic treatment apparatus 100 of this embodiment, by modifying the configuration and material of the gasket first sealing portion 144 so as to more completely seal the gap formed in the circumferential direction of the gasket 140, the apparatus of this embodiment may even possess the drip-proofness of the waterproof ratings (IPX) 2 to 8. Here, the magnetic treatment apparatus 100 of this embodiment also has protection against humidity in addition to its waterproof performance.

Moreover, the gasket 140 has the gasket second sealing portion 147. When the inlet connector inner circumferential portion 112 is press-fitted toward the direction of the inlet 120 by passing through the gasket second opening section 141b, an inlet connector inner circumferential portion side surface 116 of the inlet connector inner circumferential portion 112 will tightly engage with the gasket second sealing portion 147 without forming a gap therebetween. As a result, the gap formed between the inlet connector inner circumferential portion side surface 116 of the inlet connector inner circumferential portion 112 and the gasket opening section 141 can be completely sealed by the gasket second sealing portion 147.

Here, there are no particular limitations on the shape of the gasket second sealing portion 147 as long as it is formed into a shape protruding toward the direction of the inlet connector inner circumferential portion side surface 116 of the inlet connector inner circumferential portion 112. There may be provided multiple gasket second sealing portions 147 facing against the inner wall of the gasket opening section 141. That is, the installation location(s) and number of the gasket second sealing portions 147 can be appropriately determined in accordance with the shape of the inlet connector inner circumferential portion end surface 113 of the inlet connector inner circumferential portion 112. For example, if the inlet connector inner circumferential portion end surface 113 of the inlet connector inner circumferential portion 112 is formed into a substantially trapezoidal shape, the gasket second sealing portion(s) 147 may be respectively provided in opposing upper and lower as well as left and right locations corresponding to the trapezoidal shape i.e., there may be provided a total of four gasket second sealing portions 147. Here, there are no particular limitations on the material of the gasket 140 as long as it is a material superior in workability, water repellency, and electrostatic discharge (ESD) countermeasure performance. As the material of the gasket 140, there may be listed, for example, a natural rubber, a nitrile rubber (NBR), a butyl rubber, a silicon rubber, a fluorine rubber, EPDM, PTFE (tetrafluoroethylene resin, Teflon (registered trademark) resin), polyetheretherketone, polyphenylene sulfide, and polyoxymethylene.

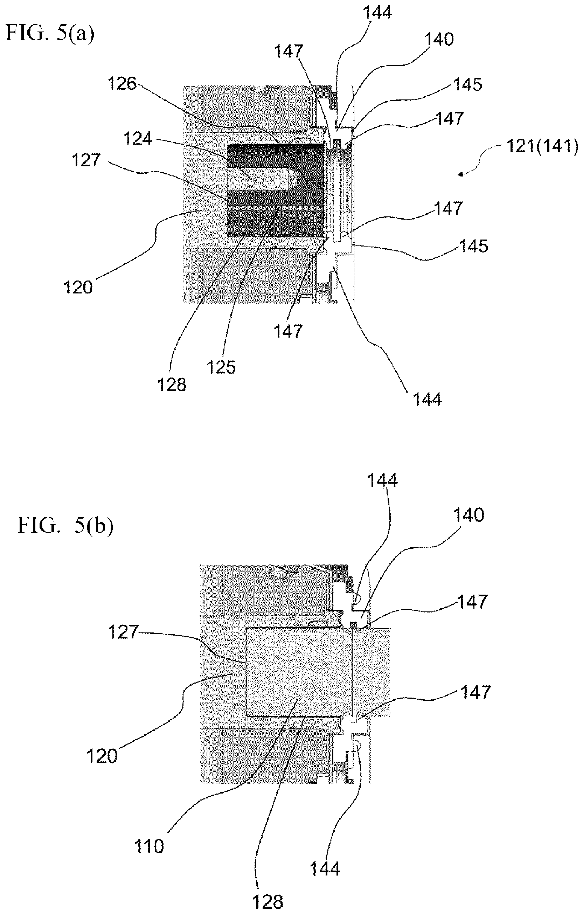

FIG.5 is a set of schematic diagrams showing states before and after connecting the inlet connector to the inlet built in the magnetic treatment apparatus of this embodiment. FIG.5(a) is a cross-sectional view showing a state before connecting the inlet connector to the inlet. As shown in FIG.5(a), the inlet 120 has, inside thereof, an inlet connector receiving portion 126 for holding the inlet connector 110 in a connected state. The inlet connector receiving portion 126 having a box shape includes an inlet bottom wall 127 that faces against the inlet opening section 121 and is located in the innermost part of the inlet connector receiving portion 126; and an inlet side wall 128 joining the inlet bottom wall 127 to the outer circumferential edge. The inlet connector receiving portion 126 is provided with inlet electric terminals 124, 125 for a power wire, a ground wire, a signal wire and the like. These electric terminals serve as electric contact sites between the apparatus main body 101 and the inlet connector 110. The gasket 140 is attached to the inlet 120 through the inlet opening section 121 and the inner substrate 130. The gasket 140 has the gasket first sealing portion 144 and the gasket second sealing portion 147.

FIG.5(b) is a cross-sectional view showing a state after connecting the inlet connector 110 to the inlet 120. As shown in FIG.5(b), once the inlet connector 110 has been inserted into the inlet connector receiving portion 126, the inlet connector inner circumferential portion 112 will engage with the inlet side wall 128. Further, as a result of allowing the inlet connector inner circumferential portion end surface 113 of the inlet connector inner circumferential portion 112 to engage with the inlet bottom wall 127, the inlet connector 110 and the inlet 120 can maintain an electrically connected state therebetween. The end surface 114 of the inlet connector outer circumferential portion 111 tightly engages with the gasket first sealing portion 144 so as to seal the circumferential direction of the inlet 120. The side surface of the inlet connector inner circumferential portion 112 tightly engages with the gasket second sealing portion 147 so as to seal the end surface direction of the inlet 120.

That is, in the case of the apparatus main body 101 of the magnetic treatment apparatus 100 of this embodiment, since the gasket second sealing portion 147 is provided in the inner part of the gasket opening section 141, the end surface direction of the inlet 120 can be sealed. In other words, in the case of the apparatus main body 101 composing the magnetic treatment apparatus 100 of this embodiment, by providing the gasket second sealing portion 147 in the inner part of the gasket opening section 141, the gap that is formed in the direction of the inlet 120 and is located between the inlet connector inner circumferential portion side surface 116 of the inlet connector inner circumferential portion 112 and the gasket opening section 141 can be completely sealed in the end surface direction, thereby effectively enabling an electrostatic discharge/surge (ESD) countermeasure and an electromagnetic susceptibility (EMS) countermeasure. Here, since the end surface direction of the inlet 120 is sealed, the apparatus main body 101 of the magnetic treatment apparatus 100 of this embodiment has an electrostatic discharge resistance even after static electricity has been discharged according to an electrostatic discharge test (ESD test). The electrostatic discharge test (ESD test) is a test for evaluating breakdown tolerance when a magnetic treatment apparatus such as a medical apparatus has been subjected to a stress imposed by static electricity. Based on models of static electricity generation, the electrostatic discharge test (ESD test) can be roughly categorized into, for example, the human body model method (HBM method), the machine model method (MM method), and the charged device model method (CDM method). The human body model method (HBM method) is a test simulating a situation where static electricity is discharged from the human body to the magnetic treatment apparatus 100, and is an electrostatic discharge test (ESD test) in which the condenser capacity is set to 100 pF and electric resistance is set to 1.5 KS2, respectively. Further, the machine model method (MM method) is a test simulating a situation where static electricity is discharged from a machine to the magnetic treatment apparatus 100, and is an electrostatic discharge test (ESD test) in which the condenser capacity is set to 200 pF and electric resistance is set to 0 S2, respectively. Furthermore, the charged device model method (CDM method) is a test simulating a situation where static electricity is discharged when the magnetic treatment apparatus 100 itself is charged, and is an electrostatic discharge test (ESD test) in which electricity is applied once. In the case of the magnetic treatment apparatus 100 of this embodiment, since the gasket second sealing portion 147 is provided in the inner part of the gasket opening section 141, the apparatus has a sufficient electrostatic discharge resistance even with regard to these electrostatic discharge tests (ESD tests). Particularly, the apparatus main body 101 of the magnetic treatment apparatus 100 of this embodiment has an air electrostatic discharge resistance of ±15 kV or higher. The electrostatic discharge that actually occurs when using a medical apparatus is air discharge taking place through the gap in the air. Here, air discharge is a form of unstable discharge as impacted by, for example, the temperature and humidity when using a medical apparatus, the approach speed when the human body is approaching the medical apparatus, the shape of a metal object worn by the human body, and the shape of a discharging object. Thus, a medical apparatus having an air electrostatic discharge resistance of ±15 kV or higher shall also have a sufficient electrostatic discharge resistance with respect to a contact discharge of about ±8 kV.

As such, in the case of the apparatus main body 101 of the magnetic treatment apparatus 100 of this embodiment, since the apparatus main body 101 has the gasket first sealing portion 144 and the gasket second sealing portion 147 on the gasket 140, the apparatus is superior in drip-proofness (IPX), electrostatic discharge/surge (ESD) countermeasure, and electromagnetic susceptibility (EMS) countermeasure. In this way, in the case of the magnetic treatment apparatus 100 of this embodiment, there is provided the gasket 140 that is interposed between the inlet opening section 121 provided at the inlet 120 and the casing opening section for inlet connector connection 104c provided at the apparatus main body 101, and is configured to allow the inlet 120 to conform with the shape of the inlet connector 110, thereby allowing the inner part of the inlet to be completely sealed. As a result, the magnetic treatment apparatus 100 of this embodiment is superior in drip-proofness (IPX), electrostatic discharge/surge (ESD) countermeasure, and electromagnetic susceptibility (EMS) countermeasure.

The invention has thus far been described based on the embodiment shown in the drawings; however, the medical apparatus of the present invention is not limited to the aforementioned embodiment, but may be appropriately modified within the scope described in the claims. That is, in the apparatus main body 101 of the magnetic treatment apparatus 100 as one example of the medical apparatus of this embodiment, appropriate modifications can be made so that the signal wave output part produces a biostimulation signal wave of a first frequency and a signal wave of a second frequency.

For example, the apparatus main body 101 of the magnetic treatment apparatus 100 of this embodiment may have a signal wave output part that produces and outputs the biostimulation signal wave of the first frequency, and have a first coil that is connected to the signal wave output part through a communication cable and is supplied with the biostimulation signal wave of the first frequency which is outputted from such signal wave output part. Further, in the apparatus main body 101 of the magnetic treatment apparatus 100 of this embodiment, the frequency of the biostimulation signal wave of the first frequency that is outputted from the signal wave output part may fluctuate within a given range with 250 MHz being the center frequency. Moreover, the apparatus main body 101 of the magnetic treatment apparatus 100 of this embodiment may also have a second coil that is supplied with the biostimulation signal wave of the second frequency which is produced by and outputted from the signal wave output part.

By setting the biostimulation signal wave of the first frequency to a biostimulation high-frequency signal wave whose center is 250 MHz, a high-frequency alternating magnetic field generated from such high-frequency signal wave will occur. Since the high-frequency alternating magnetic field generated has a high effect of inducing neurotrophic factors by activating damaged sensory cells, it is expected that the effect of alleviating the nerve disorder in an affected area can be improved. Further, by setting the biostimulation signal wave of the second frequency to a biostimulation low-frequency signal wave with a frequency of not lower than 1 KHz and not higher than 3 KHz, a low-frequency alternating magnetic field generated from such low-frequency signal wave will occur. Particularly, since the low-frequency alternating magnetic field generated is easy to reach the brain from the spinal cord dorsal horn through sensory nerves, it is expected that there can be achieved a nerve disorder alleviating effect such as a higher pain-relieving effect and relaxing effect.

### Industrial Applicability

In this way, according to the medical apparatus of the present invention, there is provided a magnetic treatment apparatus in which the sealability of the inlet portion is ensured by the gasket allowing the inlet to conform with the shape of the inlet connector, and in which the apparatus itself is therefore superior in drip-proofness (IPX), electrostatic discharge/surge (ESD) countermeasure, and electromagnetic susceptibility (EMS) countermeasure. Thus, the magnetic treatment apparatus of the present invention is industrially useful because it is an apparatus superior in drip-proofness (IPX), electrostatic discharge/surge (ESD) countermeasure, and electromagnetic susceptibility (EMS) countermeasure, and with which there can be achieved a treatment effect required to treat pain in an affected area by enhancing the output of a high-frequency signal.

The present invention has been described as above with reference to an embodiment; however, the present invention shall not be limited to the abovementioned embodiment. The configuration and particulars of the present invention may be subjected to various modifications that are comprehensible to those skilled in the art within the technical scope of the present invention.

### Reference Signs List

100 Magnetic treatment apparatus
101 Apparatus main body
102 Probe
103 Communication cable
104 Casing
104a Casing opening section
104b Casing protruding part
104c Casing opening section for inlet connector connection
105 Display
106 Battery
107 Alarm stop button
108 Power switch button
109 Plug sockets
110 Inlet connector
111 Inlet connector outer circumferential portion
112 Inlet connector inner circumferential portion
113 Inlet connector inner circumferential portion end surface
114 Inlet connector outer circumferential portion end surface
115 Inlet connector step portion end surface
116 Inlet connector inner circumferential portion side surface
120 Inlet
121 Inlet opening section
122 Inlet flange
123 Fixing tool hole
124 Inlet electric terminal
125 Inlet electric terminal
126 Inlet connector receiving portion
127 Inlet bottom wall
128 Inlet side wall
130 Inner substrate
131 Inner substrate opening section
132 Fixing tool screw
140 Gasket
141 Gasket opening section
141a Gasket first opening section
141b Gasket second opening section
142 Gasket outer edge portion
143 Fixing tool screw receiving portion
144 Gasket first sealing portion
145 Gasket front surface convex portion
146 Gasket opening section dividing part
147 Gasket second sealing portion

## Claims

1. A medical apparatus equipped with an apparatus main body having an inlet for an inlet connector to be connected thereto, comprising:
a gasket that is interposed between an inlet opening section provided at the inlet and a casing opening section for inlet connector connection provided at the apparatus main body, and is configured to allow the inlet to conform with the shape of the inlet connector.

2. The medical apparatus according to claim 1, wherein the gasket includes:
a gasket first sealing portion that tightly engages with an end surface of an outer circumferential portion of the inlet connector by pressure-contacting the inlet connector with the inlet; and
a gasket second sealing portion that is provided on an inner wall of an opening section of the gasket and tightly engages with an inner circumferential portion side surface of the inlet connector by pressure-contacting the inlet connector with the inlet.

3. The medical apparatus according to claim 2, wherein
there are provided multiple said gasket second sealing portions facing against the inner wall of the opening section of the gasket.

4. The medical apparatus according to any one of claims 1 to 3, wherein
the medical apparatus has a waterproof rating (IPX) of 1 or higher.

5. The medical apparatus according to any one of claims 1 to 4, wherein
the medical apparatus has an electrostatic discharge resistance even after static electricity has been discharged according to an electrostatic discharge test.

6. The medical apparatus according to claim 5, wherein
the electrostatic discharge resistance is an air electrostatic discharge resistance of ±15 kV or higher.

7. The medical apparatus according to any one of claims 1 to 6, further comprising
a probe formed separately from the apparatus main body and having a coil, wherein the medical apparatus treats pain in an affected area of a living body by producing a biostimulation signal wave and stimulating cells and nerves in and around the affected area as a result of irradiating the affected area with a magnetic field for affected area stimulation that is generated at the coil via the biostimulation signal wave.
